(19) Europäisches Patentamt
European Patent Office
Office européen des brevets

(11) Veröffentlichungsnummer: **0 001 414**
**B1**

# (12) EUROPÄISCHE PATENTSCHRIFT

(45) Veröffentlichungstag der Patentschrift: **30.12.81**

(51) Int. Cl.³: **C 07 D 249/08,** A 01 N 43/64

(21) Anmeldenummer: **78100931.1**

(22) Anmeldetag: **19.09.78**

## (54) Diastereomere Formen A von Triazolyl-O,N-acetalen und ihre Verwendung als Fungizide.

(30) Priorität: **29.09.77 DE 2743767**

(43) Veröffentlichungstag der Anmeldung: **18.04.79 Patentblatt 79/8**

(45) Bekanntmachung des Hinweises auf die Patenterteilung: **30.12.81 Patentblatt 81/52**

(84) Benannte Vertragsstaaten: **BE CH DE FR GB NL**

(56) Entgegenhaltungen:
**DE-A-2 324 010**
**DE-A-2 324 424**
**DE-A-2 350 122**
**DE-A-2 455 955**
**DE-A-2 640 823**

(73) Patentinhaber: **BAYER AG, Zentralbereich Patente, Marken und Lizenzen, D-5090 Leverkusen 1, Bayerwerk (DE)**

(72) Erfinder: **Krämer, Wolfgang, Dr., Am Eckbusch 39/162, D-5600 Wuppertal 1 (DE)**
Erfinder: **Büchel, Karl Heinz, Dr., Bergerheide 62, D-5600 Wuppertal 1 (DE)**
Erfinder: **Pflugbeil, Wolf-Dietrich, Dr,, Lockfinke 10, D-5600 Wuppertal 1 (DE)**
Erfinder: **Frohberger, Paul-Ernst, Dr., Willi-Baumeister-Strasse 5, D-5090 Leverkusen 1 (DE)**
Erfinder: **Brandes, Wilhelm, Dr., Eichendorffstrasse 3, D-5653 Leichlingen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG.

# Diastereomere Formen A von Triazolyl-O,N-acetalen und ihre Verwendung als Fungizide

Die Erfindung betrifft neue diastereomere Triazolyl-O,N-acetale sowie ihre Verwendung als Fungizide.

Es ist bereits bekannt geworden, dass Diastereomerengemische von Triazolyl-O,N-acetalen sowie auch einzelne diastereomere Triazolyl-O,N-acetale allgemein fungizide Wirksamkeit aufweisen (vgl. DE-OS 2 324 010).

Es wurden nun als neue Verbindungen die Enantiomerenpaare der diastereomeren Formen A (threo-Konfiguration, s. nachfolgende Erläuterung) der Triazolyl-O,N-acetale der Formel I

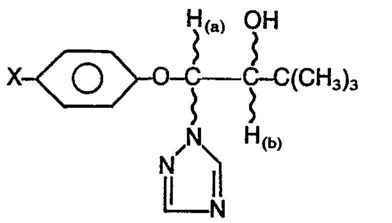
(I)

in welcher

X für Halogen oder Phenyl steht,

und wobei deren Protonen $H_{(a)}$ und $H_{(b)}$ im NMR-Spektrum kleinere Kopplungskonstanten besitzen als in der anderen diastereomeren Form (Form B, erythro-Konfiguration), gefunden. Sie weisen starke fungizide Eigenschaften auf.

Zur Erläuterung sei hierzu vermerkt:

Verbindungen mit zwei asymmetrischen Kohlenstoffatomen können in den beiden diastereomeren Formen threo und erythro vorliegen. Bei den erfindungsgemässen Verbindungen ist die Zuordnung aufgrund der noch nicht bestimmten absoluten Konfiguration nur bedingt möglich, dies wird durch die Wellenlinien in Formel I zum Ausdruck gebracht. Entsprechend wird hier in Form A und Form B unterschieden, die sich nach ihren physikalisch-chemischen Eigenschaften eindeutig charakterisieren lassen, wobei die hydrophilere Form, charakterisiert durch kleinere Kopplungskonstanten der Protonen $H_{(a)}$ und $H_{(b)}$ im NMR-Spektrum, als Form A bezeichnet wird.

Man erhält die diastereomeren Formen A der Triazolyl-O,N-acetale der Formel I, wenn man Triazolyl-ketone der Formel II

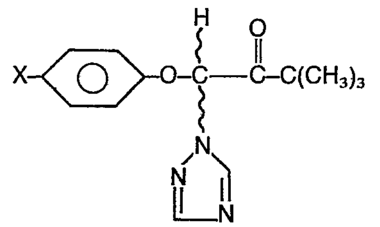
(II)

in welcher

X die oben angegebene Bedeutung hat,

mit sekundären Alkoholaten in Gegenwart eines Verdünnungsmittels stereoselektiv reduziert.

Überraschenderweise zeigen die erfindungsgemässen diastereomeren Formen A der Triazolyl-O,N-acetale der Formel I eine erheblich höhere Wirksamkeit gegen pilzliche Pflanzenkrankheiten als die aus dem Stand der Technik bekannten Diastereomerengemische von Triazolyl-O,N-acetalen. Die erfindungsgemässen Stoffe stellen somit eine wertvolle Bereicherung der Technik dar.

Aufgrund des bekannten Standes der Technik konnte keineswegs erwartet werden, dass sich die erfindungsgemässen Formen A der Triazolyl-O,N-acetale der Formel I durch sehr gute fungizide Eigenschaften auszeichnen, während die analogen Formen B dieser Verbindungen als Fungizide nur schwach aktiv sind.

Verwendet man

1-(4-Chlorphenoxy)-3,3-dimethyl-1-(1,2,4-triazol-1-yl)-butan-2-on

und Aluminiumisopropylat als Ausgangsstoffe, so kann der Reaktionsablauf durch das folgende Formelschema wiedergegeben werden:

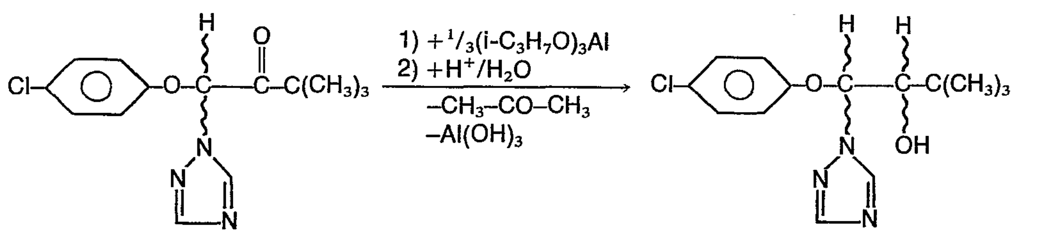


Die als Ausgangsstoffe zu verwendenden Triazolyl-ketone sind durch die Formel II allgemein definiert. In dieser Formel steht X vorzugsweise für Chlor oder Phenyl.

Zu nennen sind die folgenden erfindungsgemässen Verbindungen der Formel I, die sich durch eine besonders gute Wirksamkeit auszeichnen:

1-(4-Chlorphenoxy)-3,3-dimethyl-1-(1,2,4-triazol-1-yl)-butan-2-ol,

1-(4-Biphenylyloxy)-3,3-dimethyl-1-(1,2,4-triazol-1-yl)-butan-2-ol.

Die Ausgangsstoffe der Formel II sind bekannt (vgl. DE-OS 2 324 010 und DE-OS 2 455 955). Man erhält sie nach den dort beschriebenen Verfahren, indem man die entsprechend substituierten 1-Aryloxy-1-halogen-3,3-dimethyl-butan-2-one mit 1,2,4-Triazol, gegebenenfalls in Gegenwart eines Säurebindemittels, bei Temperaturen zwischen 60 und 120 °C umsetzt.

Die erfindungsgemässe Reduktion wird mit Hilfe von sekundären Alkoholaten durchgeführt. Hierzu gehören vorzugsweise die sekundären Alkoholate des Aluminiums, wie insbesondere Aluminiumisopropylat, Aluminium-sek.-butylat und Aluminiumcyclohexylat.

Für die erfindungsgemässe Umsetzung kommen als Verdünnungsmittel vorzugsweise inerte organische Lösungsmittel in Frage. Hierzu gehören vorzugsweise Alkoholate, wie insbesondere Isopropanol und sek. Butanol.

Die Reaktionstemperaturen können beim erfindungsgemässen Verfahren in einem grösseren Bereich variiert werden. Im allgemeinen arbeitet man zwischen etwa 80 und 120 °C, vorzugsweise bei der Siedetemperatur des Lösungsmittels.

Bei der Durchführung der erfindungsgemässen Umsetzung setzt man auf 1 Mol des Ketons der Formel II vorzugsweise 0,35 bis 1,5 Mol an sekundärem Alkoholat ein. Zur Isolierung der Verbindungen der Formel I wird das überschüssige Lösungsmittel durch Destillation im Vakuum entfernt und die entstandene Aluminium-Verbindung mit verdünnter Schwefelsäure zersetzt. Die weitere Aufarbeitung erfolgt in üblicher Weise.

Nach einer bevorzugten Ausführungsform wird zweckmässigerweise so verfahren, dass man das Triazolyl-keton der Formel II bereits in Gegenwart der entsprechenden Form A reduziert, wodurch die Stereoselektivität der Reduktion weiter zugunsten der gewünschten Form A verschoben wird. Dabei setzt man vorzugsweise auf 1 Mol des Ketons der Formel II 0,5 Mol der entsprechenden diastereomeren Form A und 0,5 Mol an sekundärem Alkoholat ein.

Die erfindungsgemässen Wirkstoffe weisen eine starke fungitoxische Wirkung auf. Sie schädigen Kulturpflanzen in den zur Bekämpfung von Pilzen notwendigen Konzentrationen nicht. Aus diesen Gründen sind sie für den Gebrauch als Pflanzenschutzmittel zur Bekämpfung von Pilzen geeignet. Fungitoxische Mittel im Pflanzenschutz werden eingesetzt zur Bekämpfung von Plasmodiophoromycetes, Oomycetes, Chytridiomycetes, Zygomycetes, Asomycetes, Basidiomycetes und Deuteromycetes.

Die erfindungsgemässen Wirkstoffe haben ein breites Wirkungsspektrum und können angewandt werden gegen parasitäre Pilze, die oberirdische Pflanzenteile befallen oder die Pflanzen vom Boden her angreifen, sowie gegen samenübertragbare Krankheitserreger. Eine besonders gute Wirksamkeit entfalten sie gegen parasitäre Pilze auf oberirdischen Pflanzenteilen.

Als Pflanzenschutzmittel können die erfindungsgemässen Wirkstoffe mit besonders gutem Erfolg zur Bekämpfung von Brandpilzen, wie z.B. gegen die Erreger des Weizensteinbrandes und des Gerstenflugbrandes, von echten Mehltaupilzen, wie beispielsweise zur Bekämpfung von Gerstenmehltau sowie von Venturia-Arten, wie gegen den Erreger des Apfelschorfs (Fusicladium dendriticum), verwendet werden.

Besonders hervorzuheben ist die teilweise systemische Wirkung der Stoffe. So gelingt es, Pflanzen gegen Pilzbefall zu schützen, wenn man den Wirkstoff über den Boden und die Wurzel den oberirdischen Teilen der Pflanze zuführt.

Als Pflanzenschutzmittel können die erfindungsgemässen Wirkstoffe zur Saatgut- oder Bodenbehandlung und zur Behandlung oberirdischer Pflanzenteile benutzt werden.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur-und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe: natürliche Gesteinsmehle, wie Kaoline, Tonerde, Talkum, Kreide, Quarz, Attapulgit, Montmorillonit oder Diatomeenerde und synthetische Gesteinsmehle, wie disperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel: nichtionogene und anionische Emulgatoren, wie Polyoxyäthylen-Fettsäure-Ester, Polyoxyäthylen-Fettalkohol-Äther z.B. Alkylaryl-polyglykol-äther, Alkylsulfonate, Alkylsulfate, Arylsulfonate sowie Eiweisshydrolysate; als Dispergiermittel: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können in den Formulierungen in Mischung mit anderen bekannten Wirkstoffen vorliegen, wie Fungiziden, Insektiziden, Akariziden, Nematiziden, Herbiziden, Schutzstoffen gegen Vogelfrass, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewandt werden. Die Anwendung geschieht in üblicher Weise, z.B. durch Giessen, Spritzen, Sprühen, Stäuben, Streuen, Trockenbeizen, Feuchtbeizen, Nassbeizen, Schlämmbeizen oder Inkrustieren.

Bei der Verwendung als Blattfungizide können die Wirkstoffkonzentrationen in den Anwendungsformen in einem grösseren Bereich variiert werden. Sie liegen im allgemeinen zwischen 0,1 und 0,00001 Gewichtsprozent. Vorzugsweise zwischen 0,05 und 0,0001%.

Bei der Saatgutbehandlung werden im allgemeinen Wirkstoffmengen von 0,001 bis 50 g je Kilogramm Saatgut, vorzugsweise 0,01 bis 10 g benötigt.

Zur Bodenbehandlung sind Wirkstoffmengen von 1 bis 100 g je cbm Boden, wie insbesondere 10 bis 200 g, erforderlich.

Die vielseitigen Verwendungsmöglichkeiten gehen aus den nachfolgenden Beispielen hervor.

Beispiel A

Saatgutbeizmittel-Test/Weizensteinbrand/Freilandversuch

(samenbürtige Mykose)

Zur Herstellung eines zweckmässigen Trockenbeizmittels verstreckt man den Wirkstoff mit einem Gemisch aus gleichen Gewichtsteilen Talkum und Kieselgur zu einer feinpulverigen Mischung mit der gewünschten Wirkstoffkonzentration.

Die Beizung erfolgt in 4 Einzelportionen von je 100 g, die auf 4 Parzellen von 5 $m^2$ Grösse zur Aussaat gebracht werden. Die angegebenen Befallsprozente werden gewonnen aufgrund der vollständigen Zählung sämtlicher kranker Ähren auf den einzelnen Parzellen und der Schätzung der Gesamtzahl aller Ähren aufgrund der Auszählung einiger Parzellen mit augenscheinlich gleicher Bestandsdichte.

Zur Wirkungsprüfung gegen Weizensteinbrand (Tilletia caries) wird Winterweizen (zentrifiziertes Saatgut) benutzt, der zuvor mit 2 g Chlamydosporen je kg Saatgut kontaminiert worden ist.

Beizung: Anfang Oktober
Aussaat: 10.–20. Oktober
Auswertung: Ende Juni bis Mitte Juli

Die Prozentzahlen kranker Ähren stützen sich auf jeweils etwa 2000 Ähren je Parzelle = insgesamt etwa 8000 Ähren je Versuchsglied.

Wirkstoffe, Wirkstoffkonzentrationen im Beizmittel, Beizmittelaufwandmenge und Anzahl der kranken Ähren gehen hervor aus der nachfolgenden Tabelle.

Tabelle A
Saatgutbeizmittel-Test/Weizensteinbrand/Freilandversuch

| Wirkstoff | Wirkstoffkonzentration im Beizmittel in Gew.-% | Beizmittelaufwandmenge in g/kg Saatgut | Anzahl steinbrandkranker Ähren in % der insgesamt ausgebildeten Ähren |
|---|---|---|---|
| ungebeizt | – | – | 90,93 |
| 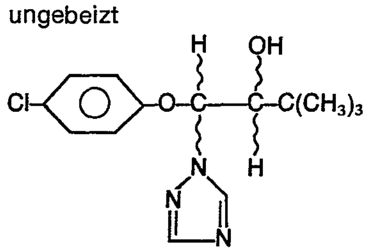 (Diastereomerengemisch) (bekannt) | 15 | 2 | 0,18 |
| Form B (Enantiomerengemisch) | 15 | 2 | 1,89 |
| Form A (1) (Enantiomerengemisch) | 15 | 2 | 0,02 |

Beispiel B

Saatgutbeizmittel-Test/Gerstenflugbrand/Freilandversuch

(samenbürtige Mykose)

Zur Herstellung eines zweckmässigen Trokkenbeizmittels verstreckt man den Wirkstoff mit einem Gemisch aus gleichen Gewichtsteilen Talkum und Kieselgur zu einer feinpulverigen Mischung mit der gewünschten Wirkstoffkonzentration.

Die Beizung erfolgt in 4 Einzelportionen von je 100 g, die auf 4 Parzellen von 5 $m^2$ Grösse zur Aussaat gebracht werden. Die angegebenen Befallsprozente werden gewonnen aufgrund der vollständigen Zählung sämtlicher kranker Ähren auf den einzelnen Parzellen und der Schätzung der Gesamtzahl aller Ähren aufgrund der Auszählung einiger Parzellen mit augenscheinlich gleicher Bestandsdichte.

Zur Wirkungsprüfung gegen Gerstenflugbrand (Ustilago nuda) wird natürlich verseuchte Sommergerste benutzt.

Beizung und Aussaat: Anfang April

Auswertung: Ende Juni bis Anfang Juli

Die Prozentzahlen kranker Ähren stützen sich auf jeweils etwa 2000 Ähren je Parzelle = insgesamt auf etwa 8000 Ähren je Versuchsglied.

Wirkstoffe, Wirkstoffkonzentrationen im Beizmittel, Beizmittelaufwandmengen und Anzahl der kranken Ähren gehen hervor aus der nachfolgenden Tabelle.

Tabelle B

Saatgutbeizmittel-Test/Gerstenflugbrand/Freilandversuch

| Wirkstoff | Wirkstoff-konzentration im Beizmittel in Gew.-% | Beizmittel-aufwandmenge in g/kg Saatgut | Anzahl flug-brandkranker Ähren in % der insgesamt ausgebildeten Ähren |
|---|---|---|---|
| ungebeizt | – | – | 29,45 |
| 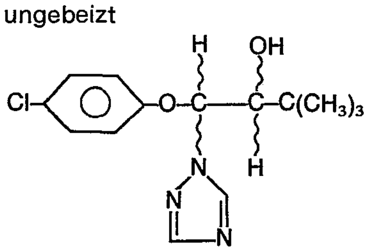 (Diastereomerengemisch) (bekannt) | 10 | 2 | 0,01 |
| Form B (Enantiomerengemisch) | 10 | 2 | 14,43 |
| Form A (1) (Enantiomerengemisch) | 10 | 2 | 0,00 |

Beispiel C

Gerstenmehltau-Test (Erysiphe graminis var. hordei)/systemisch

(pilzliche Getreidesprosskrankheit)

Die Anwendung der Wirkstoffe erfolgt als pulverförmige Saatgutbehandlungsmittel. Sie werden hergestellt durch Abstrecken des jeweiligen Wirkstoffes mit einem Gemisch aus gleichen Gewichtsteilen Talkum und Kieselgur zu einer feinpulverigen Mischung mit der gewünschten Wirkstoffkonzentration.

Zur Saatgutbehandlung schüttelt man Gerstensaatgut mit dem abgestreckten Wirkstoff in einer verschlossenen Glasflasche. Das Saatgut sät man mit 3×12 Korn in Blumentöpfe 2 cm tief in ein Gemisch aus einem Volumteil Fruhstorfer Einheitserde und einem Volumteil Quarzsand ein. Die Keimung und der Auflauf erfolgen unter günstigen Bedingungen im Gewächshaus. 7 Tage nach der Aussaat, wenn die Gerstenpflanzen ihr erstes Blatt entfaltet haben, werden sie mit frischen Sporen von Erysiphe graminis var. hordei bestäubt und bei 21–22 °C und 80–90% rel. Luftfeuchte und 16stündiger Belichtung weiter kultiviert. Innerhalb von 6 Tagen bilden sich an den Blättern die typischen Mehltaupusteln aus.

Der Befallsgrad wird in Prozenten des Befalls der unbehandelten Kontrollpflanzen ausgedrückt. So bedeutet 0% keinen Befall und 100% den gleichen Befallsgrad wie bei der unbehandelten Kontrolle. Der Wirkstoff ist um so wirksamer je geringer der Mehltaubefall ist.

Wirkstoffe, Wirkstoffkonzentrationen im Saatgutbehandlungsmittel sowie dessen Aufwandmenge und der prozentuale Mehltaubefall gehen hervor aus der nachfolgenden Tabelle.

Tabelle C
Gerstenmehltau-Test (Erysiphe graminis var. hordei)/systemisch

| Wirkstoffe | Wirkstoff-konzentration im Beizmittel in Gew.-% | Beizmittel-aufwandmenge in g/kg Saatgut | Befall in % der unbehandelten Kontrolle |
|---|---|---|---|
| ungebeizt | – | – | 100 |
| 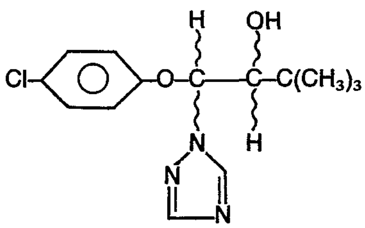 (Diastereomerengemisch) (bekannt) | 0,25 | 2 | 26,3 |
| Form B (Enantiomerengemisch) | 0,25 | 2 | 81,7 |
| Form A (1) (Enantiomerengemisch) | 0,25 | 2 | 4,2 |

Beispiel D
Fusicladium-Test (Apfel)/Protektiv
Lösungsmittel: 4,7 Gewichtsteile Aceton
Emulgator: 0,3 Gewichtsteile Alkyl-aryl-polyglykoläther
Wasser: 95 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4- bis 6-Blattstadium befinden, bis zur Tropfnässe. Die Pflanzen verbleiben 24 Stunden bei 20°C und einer relativen Luftfeuchtigkeit von 70% im Gewächshaus. Anschliessend werden sie mit einer wässrigen Konidiensuspension des Apfelschorferregers (Fusicladium dentriticum) inokuliert und 18 Stunden lang in einer Feuchtkammer bei 18 bis 20°C und 100% relativer Luftfeuchtigkeit inkubiert.

Die Pflanzen kommen dann erneut für 14 Tage ins Gewächshaus.

15 Tage nach der Inokulation wird der Befall der Sämlinge bestimmt. Die erhaltenen Boniturwerte werden in Prozent Befall umgerechnet. 0% bedeutet keinen Befall, 100% bedeutet, dass die Pflanzen vollständig befallen sind.

Wirkstoffe, Wirkstoffkonzentrationen und Ergebnisse gehen aus der nachfolgenden Tabelle hervor:

Tabelle D
Fusicladium-Test (Apfel)/Protektiv

| Wirkstoff | Befall in % bei einer Wirkstoffkonzentration von 0,0005% |
|---|---|
| 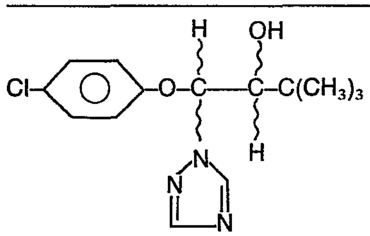 (Diastereomerengemisch) (bekannt) | 32 |
| Form B (Enantiomerengemisch) | 36 |
| Form A (1) (Enantiomerengemisch) | 5 |

Herstellungsbeispiele

Beispiel 1

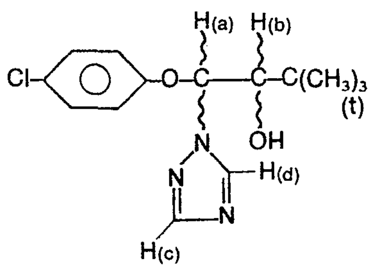

Form A
(Enantiomerengemisch)

In einen 10-Liter-Flanschkolben mit Rührer, Thermometer und einer 1m Vigreux-Kolonne werden zu 6,25 l Isopropanol und 611 g (3 Mol) Aluminium-isopropylat bei 50 °C portionenweise 1,47 kg (5 Mol)
1-(4-Chlorphenoxy)-3,3-dimethyl-1-(1,2,4-triazol-1-yl)-butan-2-on
(technische Ware, 92,8%ig) zugegeben. Nach beendeter Zugabe erhitzt man auf 98 °C Badtemperatur und destilliert innerhalb von 13 Stunden 1000 ml Lösungsmittel und entstehendes Aceton bei einer Temperatur von 72 bis 79 °C am Kolonnenkopf ab. Danach wird das Lösungsmittel unter Wasserstrahlvakuum weiter bis zur Hälfte abdestilliert. Das Reaktionsgemisch wird anschliessend unter Rühren zu einem Gemisch von 875 ml Wasser und 875 ml 10%iger Schwefelsäure gegeben. Man rührt 1,5 Stunden, saugt die entstandenen Kristalle ab, wäscht sie mehrmals mit je 300 ml Wasser aus und trocknet. Man erhält 1,125 kg (76% der Theorie)
1-(4-Chlorphenoxy)-3,3-dimethyl-1-(1,2,4-triazol-1-yl)-butan-2-ol
vom Schmelzpunkt 128–130 °C mit einem Diastereomerenanteil von 85% Form A und 11,5% Form B. Durch einmaliges Umkristallisieren aus 2,5 l Isopropanol erhält man 600 g (63% der Theorie, bezogen auf das Diastereomerengemisch) reine Form A von
1-(4-Chlorphenoxy)-3,3-dimethyl-1-(1,2,4-triazol-1-yl)-butan-2-ol
vom Schmelzpunkt 139 °C und den folgenden NMR-Daten:

$\delta[ppm]_{CD_3OD}$ $H_{(a)}$: 6,46 (J = 2 Hz), $H_{(b)}$: 3,61 (J = 2 Hz), $H_{(t)}$: 1,05, $H_{(c)}$: 8,05, $H_{(d)}$: 8,52.

In analoger Weise erhält man bei der Umsetzung von 147,8 g (0,5 Mol)
1-(4-Chlorphenoxy)-3,3-dimethyl-1-(1,2,4-triazol-1-yl)-butan-2-on
in 0,5 l Butan-2-ol mit 49,2 g (0,2 Mol) Aluminium-sek.-butylat nach 10 Stunden Reaktionszeit bei Siedetemperatur 75,8% der Theorie an reiner Form A von
1-(4-Chlorphenoxy)-3,3-dimethyl-1-(1,2,4-triazol-1-yl)-butan-2-ol
vom Schmelzpunkt 139 °C und dem oben aufgezeigten NMR-Spektrum.

Beispiel 2

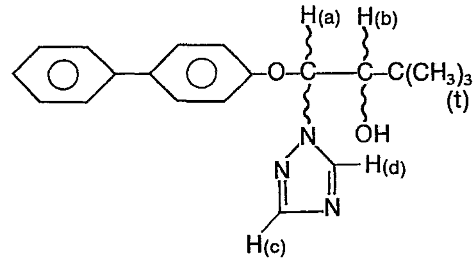

Form A
(Enantiomerengemisch)

Eine Suspension von 278 g (1,365 Mol) Aluminium-isopropylat in 2,7 l Isopropanol wird 1 Stunde bei 60 °C gerührt und anschliessend portionenweise mit 760 g (2,27 Mol)
1-(4-Biphenylyloxy)-3,3-dimethyl-1-(1,2,4-triazol-1-yl)-butan-2-on
versetzt. Man erhitzt 14 Stunden auf 80 °C, wobei über eine 30 cm Vigreux-Kolonne 260 ml Isopropanol/Aceton-Gemisch abdestilliert werden. Danach wird durch Abdestillieren des Lösungsmittels im Wasserstrahlvakuum weiter eingeengt. Der Rückstand wird mit einem Gemisch von 1000 ml Wasser, 600 ml 20%iger Schwefelsäure und 3 l Isopropanol aufgenommen, 1 Stunde bei 20 °C gerührt, mit Natriumhydrogencarbonat auf pH3 gebracht und mit 3 l Methylenchlorid extrahiert. Der Methylenextrakt wird zweimal mit je 100 ml 4%iger Natronlauge und mit 1,2 l Wasser gewaschen, über Natriumsulfat getrocknet und durch Abdestillieren des Lösungsmittels im Wasserstrahlvakuum eingeengt. Man erhält 673 g (88,3% der Theorie)
1-(4-Biphenylyloxy)-3,3-dimethyl-1-(1,2,4-triazol-1-yl)-butan-2-ol
mit einem Diastereomerenanteil von 73,2% Form A und 19,1% Form B. Durch einmaliges Umkristallisieren aus 3 l Isopropanol erhält man 349,2 g (51,9% der Theorie, bezogen auf das Diastereomerengemisch) reine Form A von
1-(4-Biphenylyloxy)-3,3-dimethyl-1-(1,2,4-triazol-1-yl)-butan-2-ol
vom Schmelzpunkt 136–137 °C und mit den folgenden NMR-Daten:

$\delta[ppm]_{CD_3OD}$ $H_{(a)}$: 6,6 (J = 2 Hz), $H_{(b)}$: 3,7 (J = 2 Hz), $H_{(t)}$: 1,16, $H_{(c)}$: 8,17, $H_{(d)}$: 8,6.

## Patentansprüche

1. Diastereomere Formen A (threo-Konfiguration) der Triazolyl-O,N-acetale der Formel I

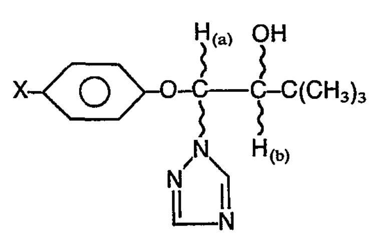

(I)

in welcher
X für Halogen oder Phenyl steht,
dadurch gekennzeichnet, dass die Protonen $H_{(a)}$ und $H_{(b)}$ im NMR-Spektrum kleinere Kopplungskonstanten besitzen als in der anderen diastereomeren Form (Form B, erythro-Konfiguration).

2. Verwendung von diastereomeren Formen A der Triazolyl-O,N-acetale gemäss Anspruch 1 zur Bekämpfung von Pilzen.

3. Fungizide Mittel, gekennzeichnet durch einen Gehalt an mindestens einer diastereomeren Form A der Triazolyl-O,N-acetale gemäss Anspruch 1.

4. Verfahren zur Herstellung von fungiziden Mitteln, dadurch gekennzeichnet, dass man diastereomere Formen A der Triazolyl-O,N-acetale gemäss Anspruch 1 mit Streckmitteln und/oder oberflächenaktiven Mitteln vermischt.

**Claims**

1. Diastereomeric forms A (threo configuration) of the triazolyl-O,N-acetals of the formula

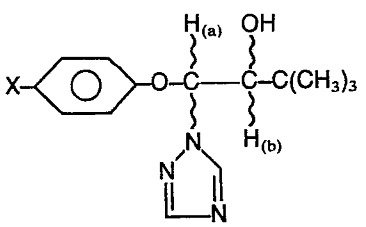
(I)

in which
X represents halogen or phenyl,
characterized in that the protons $H_{(a)}$ and $H_{(b)}$ have lower coupling constants in the NMR spectrum than in the other diastereomeric form (form B, erythro configuration).

2. The use of diastereomeric forms A of the triazolyl-O,N-acetals according to Claim 1 for combating fungi.

3. Fungicidal agents, characterised in that they contain at least one diastereomeric form A of the triazolyl-O,N-acetals according to Claim 1.

4. Process for the preparation of fungicidal agents, characterised in that diastereomeric forms A of the triazolyl-O,N-acetals according to Claim 1 are mixed with extenders and/or surface-active agents.

**Revendications**

1. Formes diastéréoisomères A (configuration thréo) des triazolyl-O,N-acétals de formule

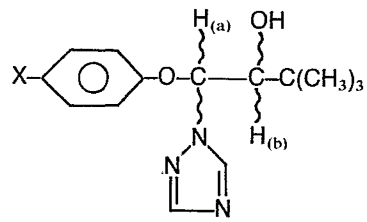
(I)

dans laquelle
X représente un halogène ou le groupe phényle, caractérisées en ce que les protons $H_{(a)}$ et $H_{(b)}$ possèdent dans le spectre de RMN des constantes de couplage plus faibles que dans l'autre forme diastéréoisomère (forme B, configuration érythro).

2. Utilisation des formes diastéréoisomères A des triazolyl-O,N-acétals selon la revendication 1 pour combattre les mycètes.

3. Produit fongicide caractérisé en ce qu'il contient au moins une forme diastéréoisomère A des triazolyl-O,N-acétals selon la revendication 1.

4. Procédé de préparation de produits fongicides caractérisé en ce que l'on mélange les formes diastéréoisomères des triazolyl-O,N-acétals selon la revendication 1 avec des diluants et/ou des agents tensioactifs.